# EUROPÄISCHE PATENTSCHRIFT

(11) **EP 1 737 859 B1**
(45) Veröffentlichungstag und Bekanntmachung des Hinweises auf die Patenterteilung: **02.12.2009**
(21) Anmeldenummer: 05716236.4
(22) Anmeldetag: 19.03.2005
(51) Int. Cl.: C07D 471/04, A61K 31/4709, A61P 31/04

(54) **NEUE KRISTALLINE FORM VON 8-CYAN-1-CYCLOPROPYL-7-(1S,6S-2,8-DIAZABICYCLO¬4.3.0 NONAN-8-YL)-6-FLUOR-1,4-DIHYDRO-4-OXO-3-CHINOLINCARBONSÄURE**
NOVEL CRYSTALLINE FORM OF 8-CYANO-1-CYCLOPROPYL-7-(1S,6S-2,8-DIAZABICYCLO¬4.3.0 NONAN-8-YL)-6-FLUORO-1,4-DIHYDRO-4-OXO-3-QUINOLINE CARBOXYLIC ACID
NOUVELLE FORME CRISTALLINE D'ACIDE 8-CYAN-1-CYCLOPROPYL-7-(1S,6S-2,8-DIAZABICYCLO¬4.3.0 NONAN-8-YL)-6-FLUOR-1,4-DIHYDRO-4-OXO-3-CHINOLINCARBOXYLIQUE

(30) Priorität: 01.04.2004 DE 102004015981
(43) Veröffentlichungstag der Anmeldung: 03.01.2007
(73) Patentinhaber: Bayer Animal Health GmbH, 51368 Leverkusen (DE)
(72) Erfinder: RAST, Hubert, 51381 Leverkusen (DE); HEEP, Iris, 50859 Köln (DE); GRUNENBERG, Alfons, 41539 Dormagen (DE); HALLENBACH, Werner, 40789 Monheim (DE); BENET-BUCHHOLZ, Jordi, 43893 Altafulla (ES)
(86) Internationale Anmeldenummer: PCT/EP2005/002953
(87) Internationale Veröffentlichungsnummer: WO 2005/097789

(56) Entgegenhaltungen:
- WO-A-00/31075
- WO-A-00/31076
- WO-A-00/31077
- WO-A-00/52009
- WO-A-00/52010
- WO-A-97/31001

## Beschreibung

Die vorliegende Erfindung betrifft das Trihydrat von Pradofloxacin, Verfahren zu seiner Herstellung sowie dieses enthaltende antibakterielle Mittel.

Die 8-Cyan-1-cyclopropyl-7-(1S,6S-2,8-diazabicyclo[4.3.0]nonan-8-yl)-6-fluor-1,4-dihydro-4-oxo-3-chinolincarbonsäure der Formel (I) soll im Folgenden mit ihrem INN (International Non-proprietary Name) als Pradofloxacin bezeichnet werden.

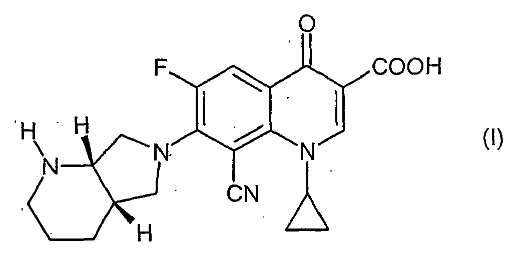

Pradofloxacin ist bekannt aus WO 97/31001 Es wird danach hergestellt durch Umsetzung von 7-Chlor-8-cyan-1-cyclopropyl-6-fluor-1,4-dihydro-4-oxo-3-chinolinearbonsäure mit (1S,6S)-2,8-Diazabicyclo[4.3.0]nonan in einem Gemisch aus Dimethylformamid und Acetonitril in Gegenwart einer Hilfsbase. Nach Versetzen mit Wasser wird Pradofloxacin mit Dichlormethan aus Wasser extrahiert und durch Entfernen des Extraktionsmittels isoliert. Man erhält dabei ein Pulver, das keine eindeutige Kristallmodifikation aufweist. Für die Herstellung von Arzneimitteln ist jedoch Voraussetzung, dass für einen Wirkstoff, der in verschiedenen Kristallmodifikationen vorliegen kann, eindeutig angegeben wird, in welcher Kristallmodifikation er zur Herstellung des Mittels eingesetzt wird.

Das z.T. amorphe Pulver, das nach dem oben skizzierten Herstellverfahren erhalten wird, ist zudem hygroskopisch. Amorphe Feststoffe, und erst recht hygroskopische Feststoffe, sind in der galenischen Verarbeitung jedoch schlecht handzuhaben, da sie beispielsweise geringe Schüttdichten und mangelhafte Fließeigenschaften aufweisen. Außerdem sind zur Handhabung hygroskopischer Feststoffe spezielle Arbeitstechniken und Einrichtungen erforderlich, um reproduzierbare Ergebnisse, z.B. bezüglich des Wirkstoffgehaltes oder der Stabilität in den produzierten Festformulierungen zu erhalten.

Definierte kristalline Formen von Pradofloxacin sind bereits bekannt: Modifikation A (WO 00/31075), Modifikation B (WO 00/31076), Modifikation C (WO 00/52009) und Modifikation D (WO 00/52010) sowie das Semi-Hydrochlorid (WO 00/31077).

Wirkstoffe für Arzneimittel sollten in Formen vorliegen, die auch unter ungünstigen Lagerbedingungen, wie erhöhte Temperatur und Luftfeuchtigkeit stabil sind. Veränderungen z.B. der Kristallstruktur sind unerwünscht, da sich damit oft auch wichtige Eigenschaften wie z.B. die Wasserlöslichkeit verändern. Grundsätzlich sucht man daher nach thermodynamisch stabilen kristallinen Formen eines Wirkstoffs.

Der Erfindung liegt daher die Aufgabe zugrunde, eine thermodynamisch stabile, definierte kristalline Form von Pradofloxacin herzustellen, die aufgrund ihrer Eigenschaften für galenische Formulierungen geeignet ist.

Überraschenderweise wurde nun das thermodynamisch sehr stabile bislang nicht bekannte Pradofloxacin-Trihydrat gefunden.

Gegenstand der Erfindung ist daher Pradofloxacin-Trihydrat, es kann mit der folgenden Formel (II) dargestellt werden:

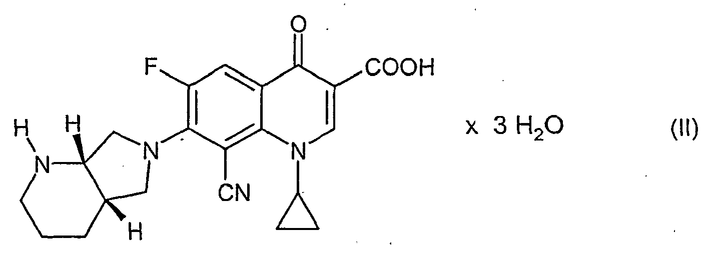

Das Pradofloxacin-Trihydrat weist ein Röntgen-Pulverdiffraktogramm mit den in der folgenden Tabelle 1 angegebene Reflexlagen ( 2 Theta) hoher und mittlerer Intensität (> 30% relative Intensität) auf.

**Tabelle 1:**

| Reflexlagen mittlerer und hoher Intensität (I_{Rei} > 30%) von Pradofloxacin-Trihydrat: |
|---|
| 2 θ(2 Theta) |
| 10.6230 |
| 14.1386 |
| 18.4032 |
| 20.9422 |
| 22.5604 |
| 22.8420 |
| 24.5165 |
| 25.8426 |
| 26.4972 |
| 26.8759 |
| 27.1231 |

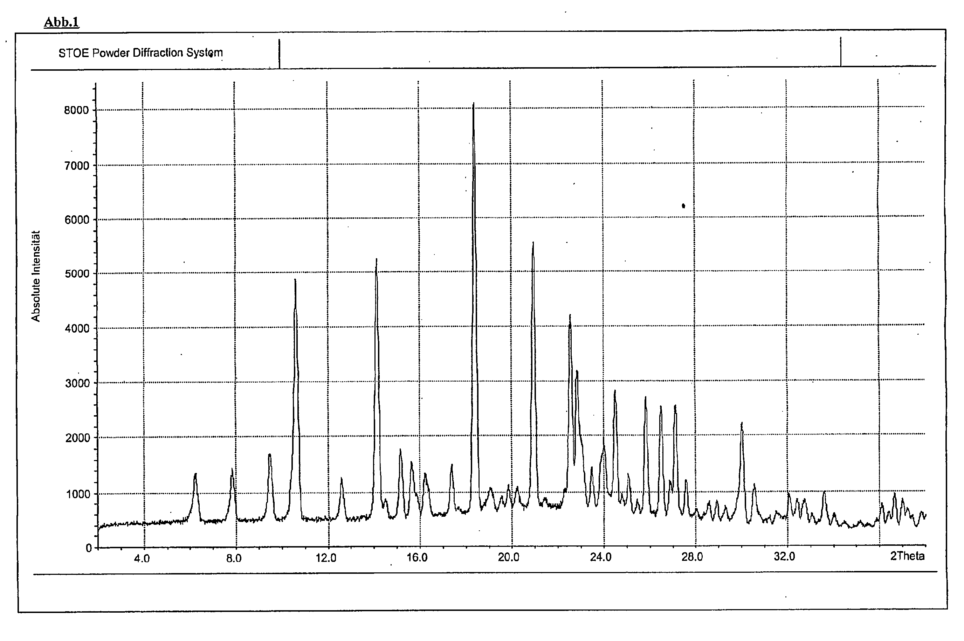

Das Pulver-Röntgendiffraktogramm von Pradofloxacin-Trihydrat ist in der Abbildung 1 wiedergegeben.

Weiterhin konnte das Pradofloxacin-Trihydrat durch Röntgenstrukturanalyse eines Einkristalls charakterisiert werden. Charakteristische Daten sind:

| Kristallsystem | Monoklin | |
|---|---|---|
| Raumgruppe | *P*2₁ | |
| Abmessungen der Elementarzelle | a = 12.4790(18)Å | α= 90°. |
| | b = 12.1275(18)Å | β= 111.009(6)°. |
| | c = 15.010(2) Å | γ = 90°. |
| Volumen | 2120.6(5) Å³ | |

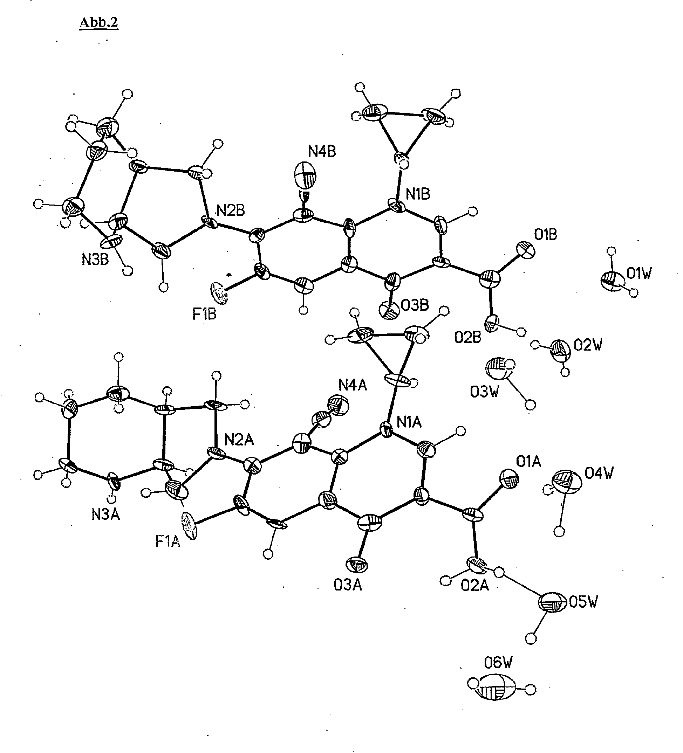

Die Struktur im Kristallgitter ist in Abb. 2 dargestellt.

Pradofloxacin-Trihydrat kann nach den folgenden Verfahren hergestellt werden:
Eine Lösung von Pradofloxacin in einem polar aprotischen Lösungsmittel wird auf eine Temperatur von 50°C oder mehr erwärmt und dann mit Wasser versetzt, das Impfkristalle von Pradofloxacin-Trihydrat enthält. '

Die Lösung in dem polar aprotischen Lösungsmittel wird vozugsweise mindestens in das gleiche Volumen Wasser gegeben, besonders bevorzügt in das 2- bis 4fache Volumen. Es kann vorteilhaft sein das erhaltene Gemisch weiter zu erwärmen auf eine Temperatur im Bereich von 50°C bis zum Siedepunkt.

Das verwendete polar aprotische Lösungsmittel sollte in ausreichendem Maße mit Wasser mischbar sein, bevorzugte Beispiele sind Dimethylformamid (DMF), Acetonitril, Propionitril und insbesondere N-Methylpyrrolidon (NMP). Auch Gemische dieser Lösungsmittel sind einsetzbar.

Alternativ kann man Pradofloxacin zusammen mit einer geringen Menge Pradofloxacin-Trihydrat in Wasser erwärmen, und zwar vorzugsweise auf eine Temperatur im Bereich 50 bis 100°C.

Weiterhin kann das Pradofloxacin-Triliydrat auch durch Umfällung über die Salze erhalten werden, wobei man zweckmäßigerweise beim Neutralisieren Prado'floxaein-Trihydrat-Impfkristalle zugibt.

Vorzugsweise wird das Pradofloxacin beim Umfällen in einer geeigneten Säure in Gegenwart von Wasser gelöst. Dann wird die Lösung mit einer Base auf pH 7 neutralisiert und die Impfkristalle werden zugegeben.

Bei allen Verfahren fällt das Pradofloxacin-Trihydrat - gegebenenfalls nach Abkühlen (z.B. auf Raumtemperatur) - als Feststoff aus.

Impfkristalle lassen sich falls erforderlich herstellen, indem man eine Probe von Pradofloxacin der Modifikation B längere Zeit bei einer Luftfeuchtigkeit von mindestens 97 % aufbewahrt, und zwar üblicherweise bei Raumtemperatur.

Pradofloxacin-Trihydrat ist überraschend stabil und wandelt sich auch bei längerer Lagerung nicht in andere Kristallformen um. Darüber hinaus zeigt das Pradofloxacin-Trihydrat keine Tendenz zur Aufnahme von weiterem Wasser aus der Luft. Schließlich lässt es sich durch Kristallisation auf einfachem Wege reinigen. Es ist aus diesen Gründen hervorragend zur Herstellung von Arzneimittelformulierungen geeignet, insbesondere solchen, in denen der Wirkstoff als Feststoff vorliegt. Durch seine Stabilität verleiht es diesen Formulierungen die gewünschte lang andauernde Lagerstabilität. Mit dem Pradofloxacin-Trihydrat können damit definiert und gezielt stabile Zubereitungen von Pradofloxacin hergestellt werden.

Pradofloxacin-Trihydrat ist hervorragend gegen pathogene Bakterien auf dem Gebiet der Human-oder Tiermedizin wirksam. Die Wirkung des Pradofloxacin-Trihydrats und damit auch sein breites Einsatzgebiet entspricht derjenigen von Pradofloxacin.

Das Röntgen-Pulverdiffraktogranun zur Charakterisierung des Pradofloxacin-Trihydr ats wurde mit einem Transmissions-Diffraktometer STADI-P (CuK_{α}-Strahlung) mit ortsempfindlichen Detektor (PSD2) der Firma Stoe erhalten.

Die Röntgenstrukturanalyse des Einkristalls wurde mit einem Siemens P4 Diffraktometer, ausgerüstet mit einem SMART-CCD-1000 Flächendetektor, einer rotierenden Anode (MACScience Co.) mit MoK-Strahlung, einem Graphitmonochromator und einer Siemens Tieftemperaturvoirichtung LT2 (T = -120°C) erhalten.

Die folgenden Beispiele illustrieren die Erfindung ohne sie einzuschränken. Die in den folgenden Beispielen verwendeten Bedingungen sind besonders bevorzugt.

### Beispiele

### Beispiel A

### Umkristallisation aus NMP/Wasser

A.1 120 g Pradofloxacin werden in 960 ml peroxidfreiem N-Methylpyrrolidon (NMP) auf 75°C erwärmt. Diese Lösung wird, über einen Faltenfilter, in 2880 ml Wasser gegossen, welches mit Pradofloxacin-Trihydrat angeimpft wurde. Man lässt ohne Rühren auf Raumtemperatur kommen und einen Tag bei Raumtemperatur stehen. Der Feststoff wird abgesaugt, zweimal mit je 100ml Wasser gewaschen und an der Luft getrocknet.

Ausbeute: 115,73 g 84,9 % d.Th.

A.2 20 g Pradofloxacin werden in 90 ml peroxidfreiem NMP auf 75°C erwärmt. Danach werden 270 ml Wasser zugegeben und weiter bis auf 100°C erhitzt. Die entstandene Lösung wird noch 15 Minuten bei dieser Temperatur gehalten, dann etwas abgekühlt und mit Pradofloxacin-Trihydrat angeimpft. Zur Kristallisation wird über Nacht stehen gelassen. Der Feststoff wird abgesaugt, zweimal mit etwas Wasser gewaschen und an der Luft getrocknet.

Ausbeute: 20,44 g 89,9 % d.Th.

In allen Fällen wurde nach den Röntgen Pulveraufnahmen das Pradofloxacin-Trihydrat erhalten.

### Beispiele B

### Erhitzen in reinem Wasser

5 g Pradofloxacin und 100 mg Pradofloxacin-Trihydrat werden zur angegebenen Menge Wasser gegeben und 3 Stunden auf die angegebene Temperatur erhitzt.

**Tabelle 2: Modifikationsumwandlung durch Erhitzen in Wasser**

| **Versuch** | **Ausbeute** | **Wassermenge** | **Bedingungen** |
|---|---|---|---|
| B.1 | 91 % | 25 ml | 85 °C |
| B.2 | 93 % | 50 ml | 85 °C |
| B.3 | 92 % | 100 ml | 85 °C |

In allen Fällen wurde nach Röntgenpulveraufnahmen Pradofloxacin-Trihydrat erhalten.

### Beispiel C:

### Umfällung über Salz

**Tabelle 3: Umfällung von Pradofloxacin**

| Versuch | Säure | Menge | Ausbeute | Bemerkung |
|---|---|---|---|---|
| | | (mmol) | | |
| | | | % | |
| C.1 | Schwefelsäure | 6 | 93,5 | Niederschlag im Sauren |
| C.2 | Essigsäure | 6 | 92 | |
| C.3 | Ameisensäure | 6 | 81,7 | |
| C.4 | Schwefelsäure | 3 | 94,2 | Niederschlag im Sauren |
| C.5 | Essigsäure | 6 | 89,8 | bei 60 °C gefällt und 2 h getempert. |

Es wird jeweils die angegebene Menge an Säure in 12 ml Wasser gelöst, 2,4 g (6 mmol) Pradofloxacin zugegeben, 15 Minuten gerührt und anschließend mit konz. Ammoniak-Lösung auf pH 7,0 neutralisiert. Sobald die Lösung sich eintrübt werden Impfkristalle von Pradofloxacin-Trihydrat zugegeben. Es wird über Nacht bei Raumtemperatur nachgeführt, dann abgesaugt und an der Luft getrocknet.

In allen Fällen wurde nach Röntgenpulveraufnahnen Pradofloxacin-Trihydrat erhalten.

## Patentansprüche

1. Pradofloxacin- Trihydrat.

2. Arzneimittel, enthaltend Pradofloxacin-Trihydrat gemäß Anspruch 1.

3. Verwendung von Pradofloxacin-Trihydrat gemäß Anspruch 1 zum Herstellung von Arzneimitteln.

4. Verwendung gemäß Anspruch 3 zur Herstellung von Arzneimitteln zur Behandlung bakterieller Erkrankungen.

5. Pradofloxacin-Trihydrat zur Verwendung in Arzneimitteln.

6. Pradofloxacin-Trihydrat zur Verwendung zur Behandlung bakterieller Erkrankungen.

## Claims

1. Pradofloxacin trihydrate.

2. Medicament comprising pradofloxacin trihydrate according to Claim 1.

3. Use of pradofloxacin trihydrate according to Claim 1 for preparing medicaments.

4. Use according to Claim 3 for preparing medicaments for treating bacterial disorders.

5. Pradofloxacin trihydrate for use in medicaments.

6. Pradofloxacin trihydrate for use for treating bacterial disorders.

## Revendications

1. Trihydrate de pradofloxacine.

2. Médicament contenant du trihydrate de pradofloxacine selon la revendication 1.

3. Utilisation de trihydrate de pradofloxacine selon la revendication 1 pour la préparation de médicaments.

4. Utilisation selon la revendication 3 pour la préparation de médicaments destinés au traitement d'affections bactériennes.

5. Trihydrate de pradofloxacine destiné à être utilisé dans des médicaments.

6. Trihydrate de pradofloxacine destiné à être utilisé pour le traitement d'affections bactériennes.
